# EUROPEAN PATENT APPLICATION

(11) **EP 3 791 916 A1**
(43) Date of publication of application: **17.03.2021**
(21) Application number: 18917661.3
(22) Date of filing: 09.05.2018
(51) Int. Cl.: A61M 25/09, A61B 5/01, A61B 5/0215

(54) **MEDICAL-USE TUBE**

(71) Applicant: ASAHI INTECC CO., LTD., Seto-shi, Aichi 489-0071 (JP)
(72) Inventor: SUGITA Shuji, Seto-shi, Aichi 489-0071 (JP); SAWAI Akira, Seto-shi, Aichi 489-0071 (JP)
(74) Representative: Winter, Brandl, Fürniss, Hübner, Röss, Kaiser, Polte - Partnerschaft mbB
(86) International application number: PCT/JP2018/017886
(87) International publication number: WO 2019/215827

(57) **Abstract**

<Problem to be solved>

To provide a medical tube which can be used in a medical instrument including a guide wire, secures torquability, enhances flexibility, and can disperse stress to prevent rupture, without becoming inoperable even when curved.

<Means for solving the problem>

A medical tube 3 includes an inner tube shaft 2 which has a base end portion 8 and a tip end portion 4 which is formed on a tip end side of the base end portion 8, has an outer diameter smaller than an outer diameter of the base end portion 8, and has on a surface thereof a slit 6 extending in a θ2 direction with respect to a longitudinal direction of the medical tube 3; and an outer tube shaft 5 which covers an outer periphery of the tip end portion 4 of the inner tube shaft 2 and has on a surface thereof a slit 7 extending in a θ1 direction crossing the θ2 direction.

## Description

### TECHNICAL FILED

The present invention relates to a medical tube for use in medical fields.

### BACKGROUND ART

Traditionally, various guide wires have been proposed for guiding a catheter and the like which is inserted, when used, into a tubular organ such as a blood vessel, gastrointestinal tract, and ureter, and body issues for the purposes of treatment and laboratory tests. Guide wires usually require blood-vessel followability for allowing them to follow along a curved blood vessel of a patient; torquability for allowing the tip ends of them to rotate smoothly inside a blood vessel of a patient; and further tip-end flexibility for preventing damage to a blood vessel of a patient.

Among them, a guide wire with a pressure sensor for measuring the internal pressure of a blood vessel of a patient needs to be configured to have a lumen through which the pressure sensor and a lead wire therefor are passed, and thus its hollow tubular structure is particularly required to satisfy the aforementioned requirements.

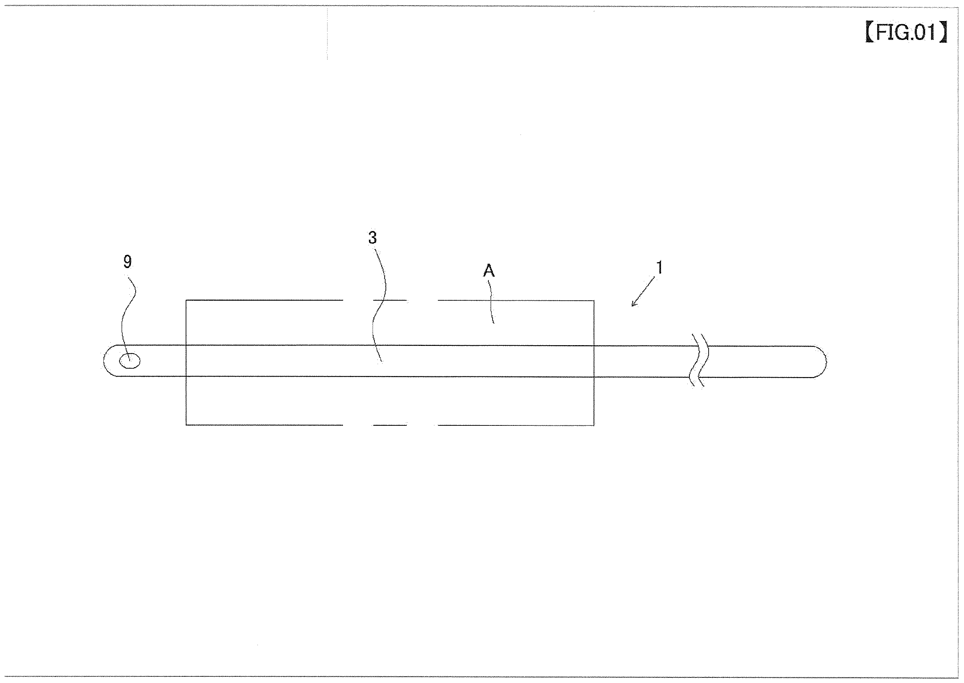

For example, Patent Document 1 describes a pressure-sensor guide wire having a pressure sensor for measuring the internal pressure of a blood vessel of a patient (see Fig. 1 and others).

The pressure-sensor guide wire 10 described in Patent Document 1 includes a proximal portion 12, an intermediate portion 14, a sensor housing portion 15, and a tip end portion 16, in which a pressure sensor 34 is arranged in the inside of the sensor housing part 15 so as to positioned in the vicinity of an opening 30 formed at the sensor housing part 15.

Moreover, a spiral cut pattern is formed in the intermediate portion 14 of the pressure-sensor guide wire 10, and an inner hypotube 20 is inserted into the intermediate portion 14 so as to be overlapped with the spiral cut pattern.

However, the pressure-sensor guide wire described in Patent Document 1 suffers from the following problem: flexibility obtained from the presence of the spiral cut pattern formed in the intermediate portion may eventually be impaired by the inner hypotube inserted into the intermediate portion, resulting in rapture when the pressure-sensor guide wire described in Patent Document 1 is curved to a large curvature.

Accordingly, an idea in that another cut pattern is formed in the inner hypotube in order to maintain flexibility has been proposed. However, this was found to suffer from the following problem: when the guide wire is curved, either an outer slit or an inner slit may enter into a girder of the other if the slits are formed arbitrarily. Therefore, the guide wire may become inoperable inside the body of a patient during the procedure.

Moreover, the pressure-sensor guide wire described in Patent Document 1 is configured such that the intermediate portion and the inner hypotube are separate entities. Disadvantageously, this may generate a gap between them during various' operations of the guide wire, resulting in impaired torquability.

### CITATION LIST

### Patent Document

Patent Document 1: Japanese Patent No. 5866371

### SUMMARY OF INVENTION

### Technical Problem

The present invention is made in order to solve the aforementioned problems in the conventional technology. An object of the present invention is to provide a medical tube which can be used in a medical instrument, including a guide wire; secure torquability; enhance flexibility, and disperse stress to prevent rupture without becoming inoperable even when curved.

### Solution to Problem

In order to solve the problems described above, a medical tube according to a first aspect of the present invention includes a first hollow shaft including a base end portion and a tip end portion, the tip end portion being formed in a tip end side of the base end portion and having an outer diameter smaller than that of the base end portion and including a first slit formed on a surface thereof, the first slit extending in a first direction; and a second hollow shaft for covering an outer periphery of the tip end portion of the first hollow shaft and including a second slit formed on a surface thereof, the second slit extending in a second direction crossing the first direction.

Further, in a second aspect of the present invention, provided is the medical tube according to the first aspect, in which the first slit formed on the first hollow shaft and the second slit formed on the second hollow shaft extend spirally.

Moreover, in a third aspect of the present invention, provided is the medical tube according to the second aspect, in which a spiral direction of the second slit formed on the second hollow shaft is opposite to a spiral direction of the first slit formed on the first hollow shaft.

Furthermore, in a fourth aspect of the present invention, provided is the medical tube according to the second or third aspect, in which the second hollow shaft includes a hollow twisted wire, the follow twisted wire including a single twisted elemental wire or multiple twisted elemental wires.

Further, in a fifth aspect of the present invention, provided is the medical tube according to any one of the first to fourth aspects, in which an outer diameter of a tip end of the second hollow shaft in a state where the second hollow shaft covers the outer periphery of the tip end portion of the first hollow shaft is smaller than that of the base end portion of the first hollow shaft.

### ADVANTAGEOUS EFFECTS OF INVENTION

According to the medical tube of the first aspect of the present invention, provided is a medical tube including a first follow shaft including a base end portion and a tip end portion, the tip end portion being formed in the tip end side of the base end portion and having an outer diameter smaller than that of the base end portion and including a first slit formed on a surface thereof, the first slit extending in a first direction; and a second hollow shaft for covering the outer periphery of the tip end portion of the first hollow shaft and including a second slit formed on a surface thereof, the second slit extending in a second direction crossing the first direction. This can allow for secured torquability, improved flexibility, and further dispersion of stress over the first hollow shaft and the second hollow shaft to prevent rupture of the medical tube without causing the medical tube inoperable even when curved.

Further, in the second aspect of the present invention, provided is the medical tube according to the first aspect, in which the first slit formed on the first hollow shaft and the second slit formed on the second hollow shaft both extend spirally. This can provide an advantageous effect of simple manufacturing of the medical tube, in addition to the advantageous effect of the medical tube according to the first aspect.

Moreover, in the third aspect of the present invention, provided is the medical tube according to the second aspect, in which the spiral direction of the second slit formed on the second hollow shaft is opposite to the spiral direction of the first slit formed on the first hollow shaft. Therefore, in addition to the advantageous effect of the second aspect, the first hollow shaft or the second hollow shaft can rotate in a tightening direction when a base end of the medical tube is rotated in either the clockwise or counter-clockwise direction, leading to improved torquability of the medical tube.

Furthermore, in the fourth aspect of the present invention, provided is the medical tube according to the second or third aspect, in which the second hollow shaft includes a hollow twisted wire, the follow twisted wire including a single twisted elemental wire or multiple twisted elemental wires. This, in addition to the advantageous effect of the medical tube according to the second or third aspect, can further improve flexibility of the tip end portion of the medical tube.

Further, in the fifth aspect of the present invention, provided is the medical tube according to any one of the first to fourth aspects, in which the outer diameter of the tip end of the second hollow shaft in a state where the second hollow shaft covers the outer periphery of the tip end portion of the first hollow shaft is smaller than that of the base end portion of the first hollow shaft. This, in addition to the advantageous effect of the medical tube according to any one of the first to fourth aspects, can further improve flexibility of the tip end portion.

### BRIEF DESCRIPTION OF DRAWINGS

Fig. 1 shows a schematic diagram of a sensor guide wire including a medical tube according to the first embodiment of the present invention.
Fig. 2 shows an enlarged external view of a portion A of the medical tube according to the first embodiment shown in Fig. 1.
Fig. 3 shows an external view and a sectional view at B-B of an outer tube shaft (the second hollow shaft) of the medical tube according to the first embodiment.
Fig. 4 shows an external view and a sectional view at C-C of an inner tube shaft (the first hollow shaft) of the medical tube according to the first embodiment.
Fig. 5 shows a longitudinal sectional view of the medical tube according to the first embodiment.
Fig. 6 shows an external view and a sectional view at D-D of an outer tube shaft (the second hollow shaft) of a medical tube according to the second embodiment.
Fig. 7 shows an external view of the medical tube according to the second embodiment.
Fig. 8 shows an external view and a sectional view at E-E of an outer tube shaft (the second hollow shaft) of a medical tube according to the third embodiment.
Fig. 9 shows an external view of the medical tube according to the third embodiment.
Fig. 10 shows an external view of a medical tube according to the fourth embodiment.
Fig. 11 shows an external view of a medical tube according to the fifth embodiment.
Fig. 12 shows an external view of a medical tube according to the sixth embodiment.

### DESCRIPTION OF EMBODIMENT

Below, the embodiments of the present invention will be described with reference to the drawings.

### First embodiment

First, the first embodiment of the present invention will be described. It is noted that the figures used for describing the present embodiment may be drawn with exaggeration for clear understanding, and may not be scaled correctly. In particular, the shapes of the slits described below are significantly enlarged for clear understanding.

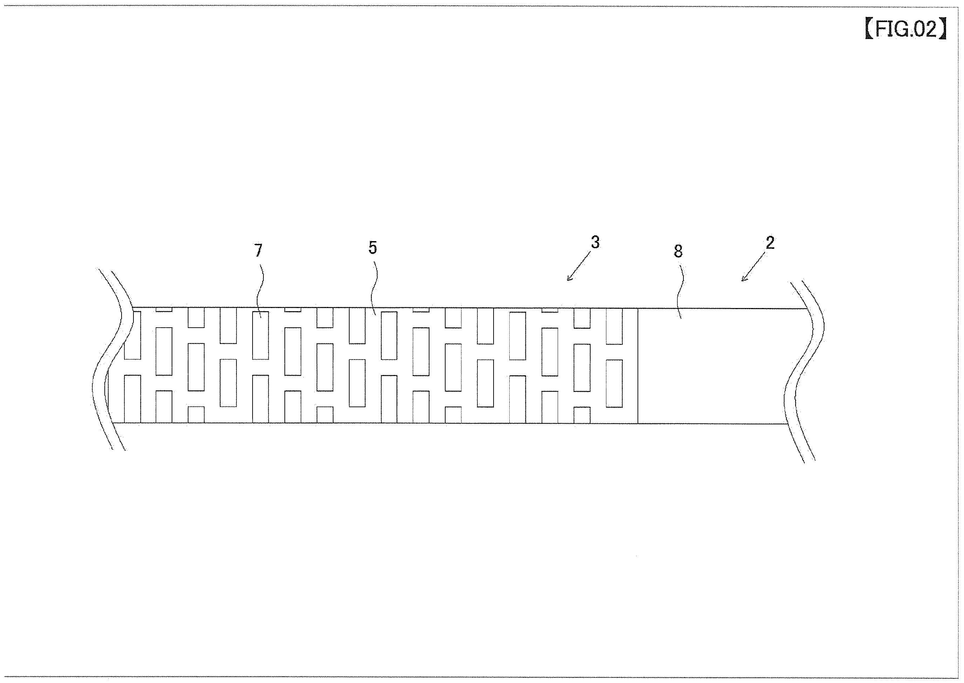

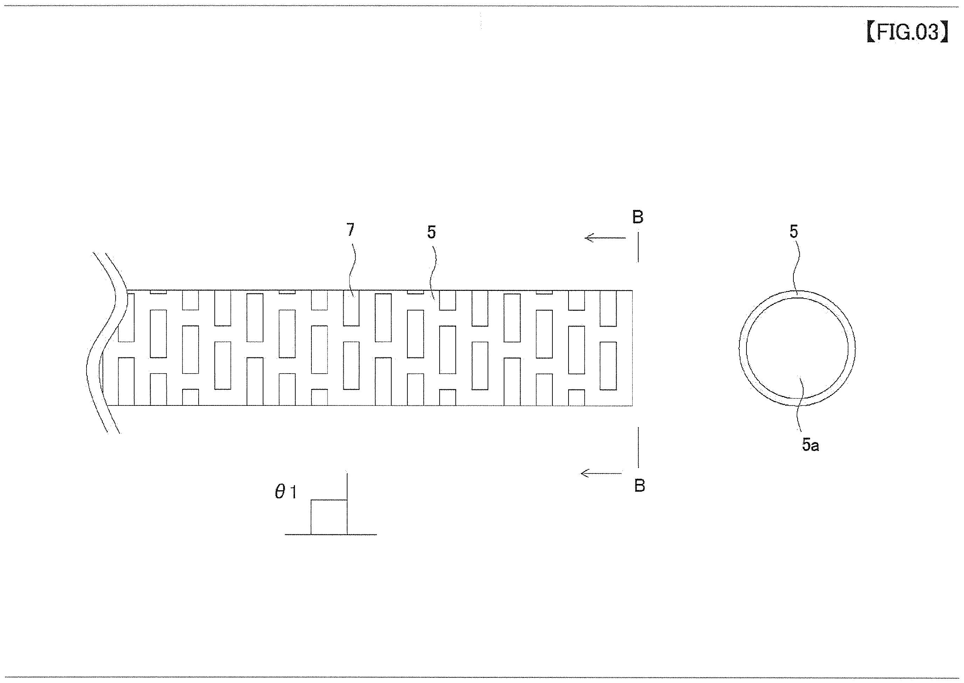

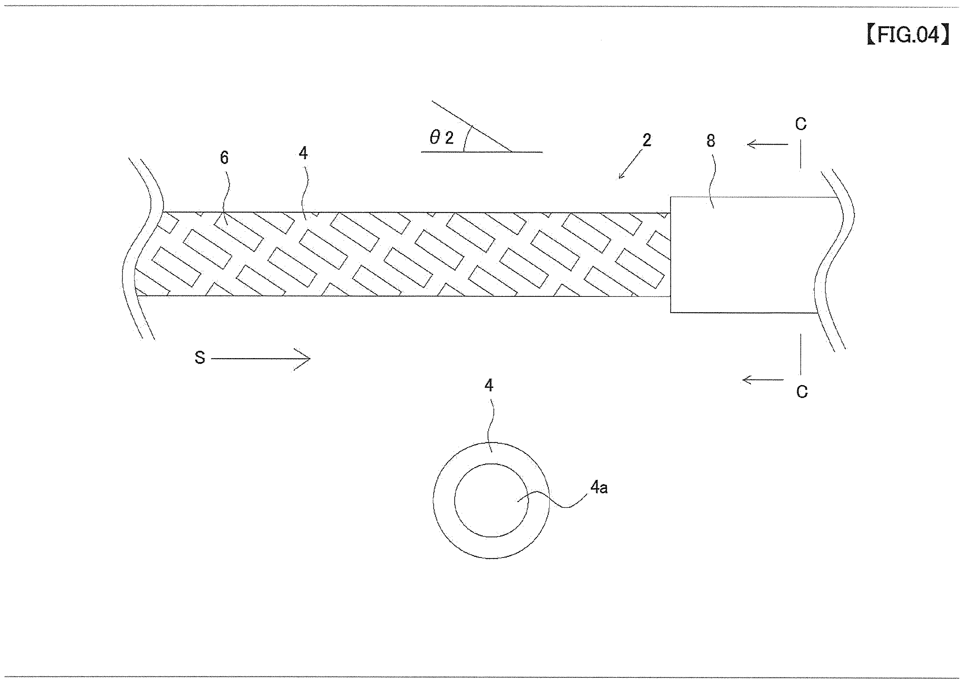

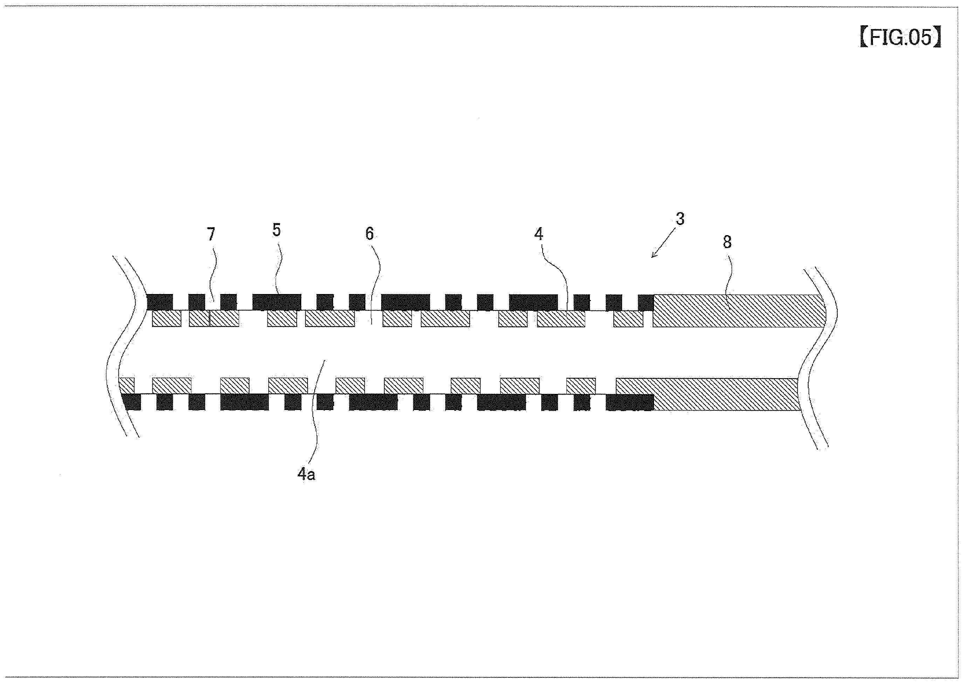

Fig. 1 shows a schematic diagram of a sensor guide wire including a medical tube according to the first embodiment of the present invention. Fig. 2 shows an enlarged external view of a portion A of the medical tube according to the first embodiment shown in Fig. 1. Fig. 3 shows an external view and a sectional view at B-B of an outer tube shaft (the second hollow shaft) of the medical tube according to the first embodiment. Fig. 4 shows an external view and a sectional view at C-C of an inner tube shaft (the first hollow shaft) of the medical tube according to the first embodiment. Fig. 5 shows a longitudinal sectional view of the medical tube according to the first embodiment.

As shown in Fig. 1, a sensor guide wire 1 according to the present embodiment includes a medical tube 3 having an elongated shape, an opening 9 formed on a tip end portion of the medical tube 3, and a sensor (not shown) arranged at the vicinity of the opening 9 in the inside of the medical tube 3. For example, the sensor guide wire 1 is inserted into a blood vessel when used. For example, a sensor and the like for measuring a blood temperature, a blood oxygen level, or a blood pressure may be used as a sensor in the sensor guide wire.

As shown in Figs. 2 to 5, the medical tube 3 includes an inner tube shaft 2 (which corresponds to the "first hollow shaft" according to an embodiment of the present invention) including a base end portion 8 and a tip end portion 4, the tip end portion 4 being formed in a tip end side of the base end portion 8; and an outer tube shaft 5 (which corresponds to the "second hollow shaft" according to an embodiment of the present invention) inserted into an outer periphery of the tip end portion 4 of the inner tube shaft 2 in an S direction (see Fig. 4).

The inner tube shaft 2 is an elongated hollow cylindrical member, and includes the base end portion 8 and the tip end portion 4, the tip end portion 4 being formed in the tip end side of the base end portion 8, in which the outer diameter of the tip end portion 4 is smaller than that of the base end portion 8, and a plurality of slits 6 (each of which corresponds to the "first slit" according to an embodiment of the present invention) are formed on a surface of the tip end portion 4 spirally in the clockwise direction toward the tip end. Further, the inner tube shaft 2 has a lumen 4a in communication with the base end portion 8 and the tip end portion 4.

It is noted that in the present embodiment, the direction in which the slits 6 extend is a θ2 direction (which corresponds to the "first direction" according to an embodiment of the present invention) relative to the longitudinal direction of the medical tube 3 as shown in Fig. 4.

The outer tube shaft 5 is an elongated hollow cylindrical member as in the inner tube shaft 2, and has an outer diameter identical to that of the base end portion 8 of the inner tube shaft 2. Further, the outer tube shaft 5 includes a plurality of slits 7 (each of which corresponds to the "second slit" according to an embodiment of the present invention) formed on a surface thereof, the slits 7 extending in a θ1 direction (which corresponds to the "second direction" according to an embodiment of the present invention) perpendicular to the longitudinal direction of the medical tube 3. Moreover, the outer tube shaft 5 has a lumen 5a into which the tip end portion 4 of the inner tube shaft 2 can be inserted.

There is no particular limitation for the materials of the inner tube shaft 2 and the outer tube shaft 5 as long as they are flexible materials having biocompatibility, for example, metals or metal compounds such as stainless steel, nickel-titanium alloys, tungsten, gold, platinum, iridium, cobalt-chromium alloys; or polyolefins such as polyethylenes, polypropylenes, ethylene-propylene copolymers, ethylene-vinyl acetate copolymers; thermoplastic resins such as soft polyvinyl chlorides; various rubbers such as silicone rubber and latex rubber; various elastomers such as polyurethane elastomers, polyamide elastomers, and polyester elastomers; crystalline plastics such as polyamides, crystalline polyethylenes, and crystalline polypropylenes. Stainless steel is used in the present invention.

The medical tube 3 according to the present embodiment includes the inner tube shaft 2 including the base end portion 8 and the tip end portion 4, the tip end portion 4 being formed in the tip end side of the base end portion 8 and having an outer diameter smaller than that of the base end portion 8 and including the slits 6 formed on a surface thereof, the slits 6 extending in the θ2 direction relative to the longitudinal direction of the medical tube 3; and the outer tube shaft 5 for covering the outer periphery of the tip end portion 4 of the inner tube shaft 2 and including the slits 7 formed on a surface thereof, the slits 7 extending in the θ1 second direction crossing the θ2 direction. Therefore, torquability can be secured by the integral formation of the base end portion 8 and the tip end portion 4 of the inner tube shaft 2. Further, flexibility can be improved by the presence of the slits 6 and the slits 7 formed on the tip end portion 4 of the inner tube shaft 2 and the outer tube shaft 5. Moreover, the direction in which the slits 6 extend is configured to cross the direction in which the slits 7 extend. This can disperse stress over the inner tube shaft 2 and the outer tube shaft 5 without causing the medical tube 3 inoperable even when the medical tube 3 is curved, thereby preventing rapture of the medical tube 3.

It is noted that in the present embodiment, the slits 6 are configured so as to extend in the θ2 direction relative to the longitudinal direction of the medical tube 3, and the slits 7 are configured so as to extend in the θ1 direction perpendicular to the longitudinal direction of the medical tube 3, but the configuration shall not be limited to this embodiment. Similar effects can be obtained as long as the slits 6 and the slits 7 are formed in directions where they cross each other.

For example, the slits 6 may be configured so as to extend in the θ1 direction particular to the longitudinal direction of the medical tube 3, and the slits 7 may be configured so as to extend in the θ2 direction relative to the longitudinal direction of the medical tube 3.

### Second embodiment

Next, the second embodiment of the present invention will be described. It is noted that the figures used for describing the present embodiment may also be drawn with exaggeration for clear understanding, and may not be scaled correctly. In particular, the shapes of the slits described below are significantly enlarged for clear understanding.

Below, the second embodiment of the present invention will be described. The sensor guide wire has an overall structure similar to that shown in Fig. 1 and an inner tube shaft 2 similar to that shown in Fig. 4. Therefore, their descriptions are omitted. The same symbols are assigned to portions common with the first embodiment, and their descriptions are omitted.

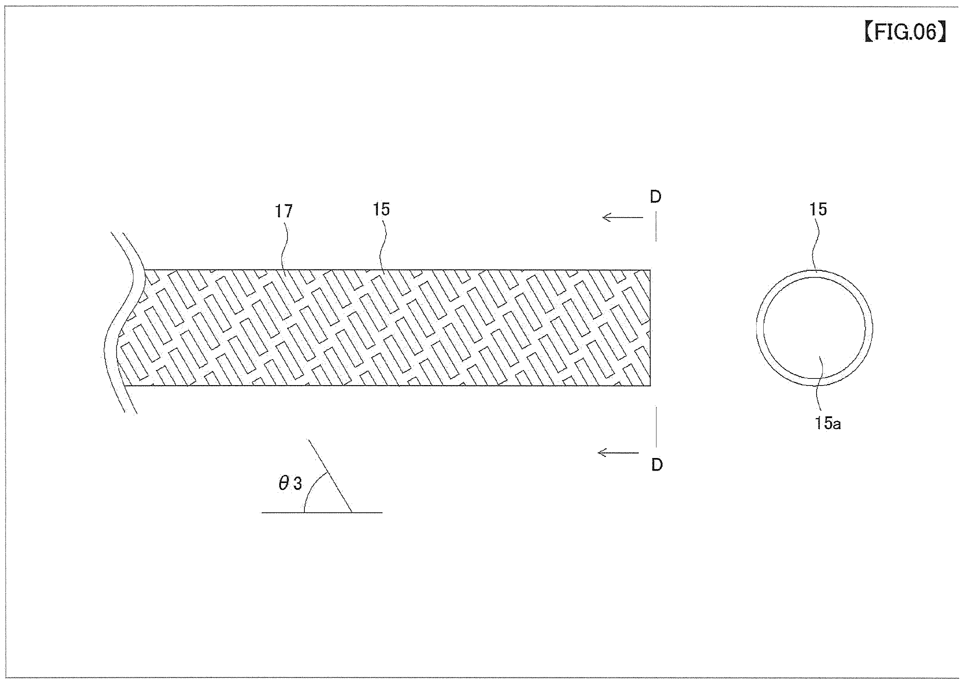

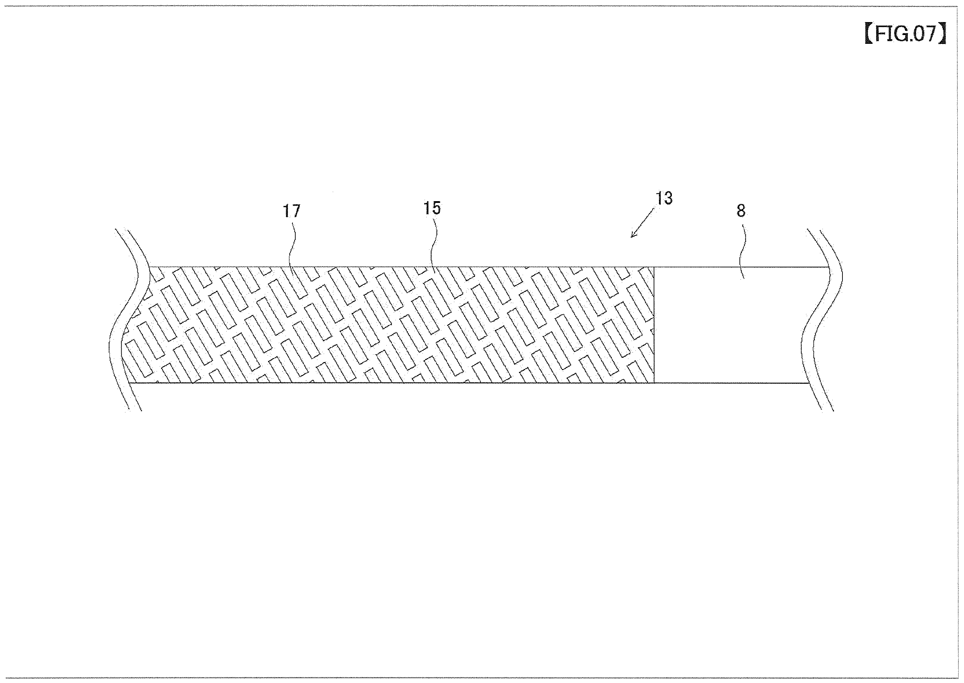

Fig. 6 shows an external view and a sectional view at D-D of an outer tube shaft (the second hollow shaft) of a medical tube of the second embodiment. Fig. 7 shows an external view of the medical tube according to the second embodiment.

As shown in Figs. 6 and 7, a medical tube 13 includes the inner tube shaft 2 (which corresponds to the "first hollow shaft" of an embodiment of the present invention) including the base end portion 8 and the tip end portion 4, the tip end portion 4 being formed in the tip end side of the base end portion 8; and an outer tube shaft 15 (which corresponds to the "second hollow shaft" of an embodiment of the present invention) inserted into the outer periphery of the tip end portion 4 of the inner tube shaft 2.

The outer tube shaft 15 is an elongated hollow cylindrical member as in the inner tube shaft 2, and has an outer diameter identical to that of the base end portion 8 of the inner tube shaft 2. Further, the outer tube shaft 15 includes a plurality of slits 17 (each of which corresponds to the "second slit" of an embodiment of the present invention) formed on a surface thereof spirally in the clockwise direction toward the tip end.

It is noted that the direction in which the slits 17 extend is a θ3 direction (which corresponds to the "second direction" according to an embodiment of the present invention. See Fig. 6.) relative to the longitudinal direction of the medical tube 13, and different from the θ2 direction of the slits 6. Further, the outer tube shaft 15 has a lumen 15a into which the tip end portion 4 of the inner tube shaft 2 can be inserted.

Moreover, the outer tube shaft 5 may be made of a similar material as those of the inner tube shaft 2 and the outer tube shaft 5.

In the medical tube 13 according to the present embodiment, the slits 6 formed on the inner tube shaft 2 and the slit 17 formed on the outer tube shaft 15 are both configured so as to extend spirally. Therefore, the medical tube 13 can be manufactured easily. Torquability can be secured by the integral formation of the base end portion 8 and the tip end portion 4 of the inner tube shaft 2. Further, flexibility can be improved by the presence of the slits 6 and the slits 17 formed on the tip end portion 4 of the inner tube shaft 2 and the outer tube shaft 15. Moreover, the direction in which the slits 6 extend is configured to cross the direction in which the slits 17 extend. This can disperse stress over the inner tube shaft 2 and the outer tube shaft 15 without causing the medical tube 13 inoperable even when the medical tube 13 is curved, thereby preventing rapture of the medical tube 13.

It is noted that in the present embodiment, the slits 6 are configured so as to extend in the θ2 direction relative to the longitudinal direction of the medical tube 13, and the slits 17 are configured so as to extend in the θ3 direction perpendicular to the longitudinal direction of the medical tube 13, but the configuration shall not be limited to this embodiment. Similar effects can be obtained as long as the slits 6 and the slits 17 are formed spirally in directions where they cross each other.

### Third embodiment

Next, the third embodiment of the present invention will be described. It is noted that the figures used for describing the present embodiment may also be drawn with exaggeration for clear understanding, and may not be scaled correctly. In particular, the shapes of the slits described below are significantly enlarged for clear understanding.

Below, the third embodiment of the present invention will be described. The sensor guide wire has an overall structure similar to that shown in Fig. 1 and an inner tube shaft 2 similar to that shown in Fig. 4. Therefore, their descriptions will be omitted. The same symbols are assigned to portions common with the first embodiment, and their descriptions are omitted.

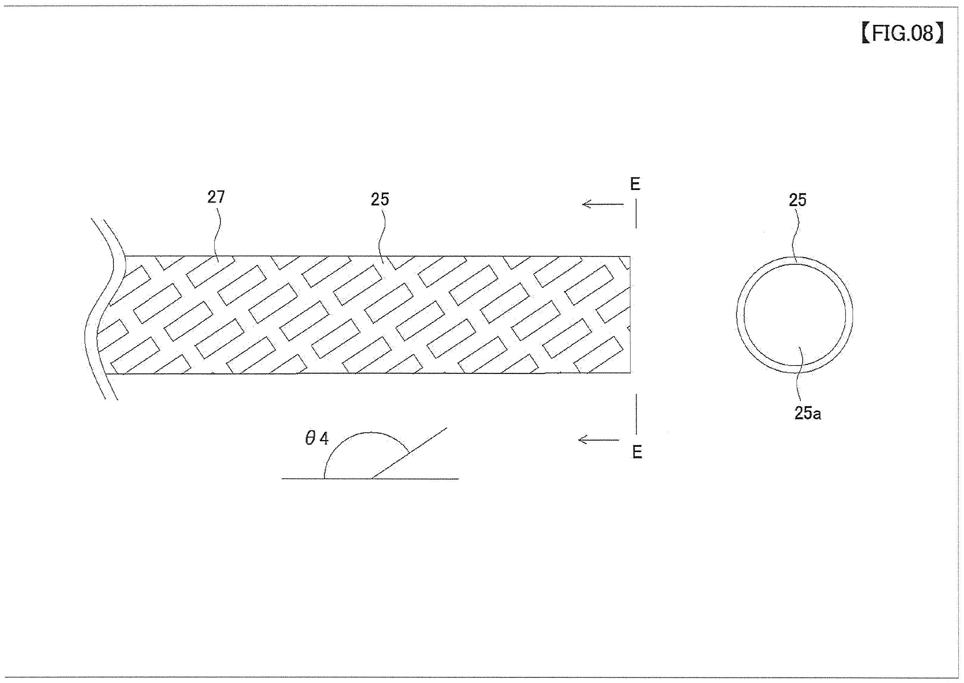

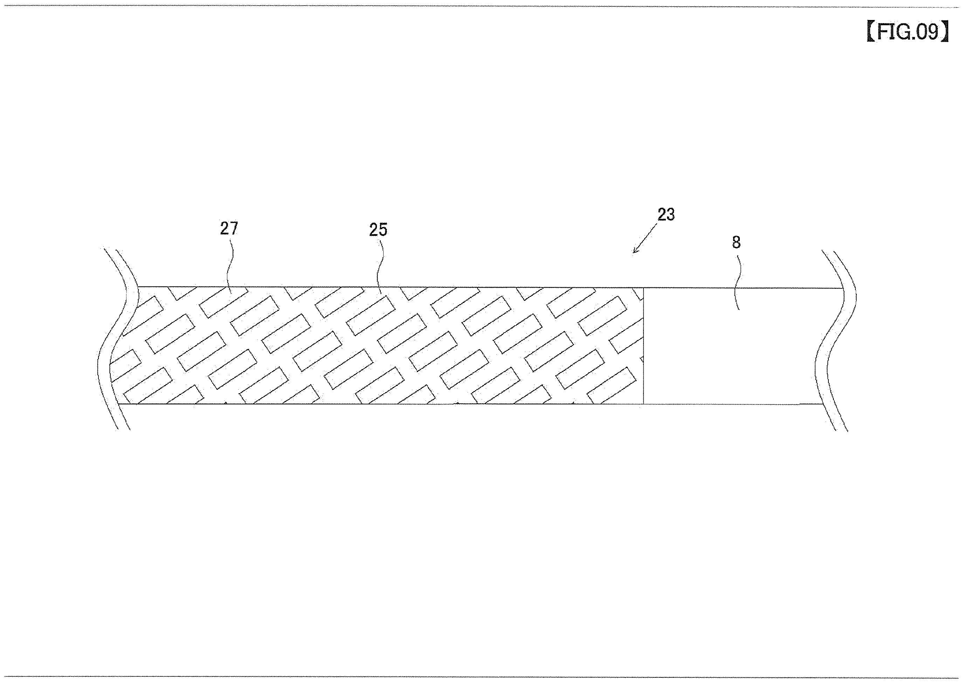

Fig. 8 shows an external view and a sectional view at E-E of an outer tube shaft (the second hollow shaft) of a medical tube according to the third embodiment. Fig. 9 shows an external view of the medical tube according to the third embodiment.

As shown in Figs. 8 and 9, a medical tube 23 includes the inner tube shaft 2 (which corresponds to the "first hollow shaft" of an embodiment of the present invention) including the base end portion 8 and the tip end portion 4, the tip end portion 4 being formed in the tip end side of the base end portion 8; and an outer tube shaft 25 (which corresponds to the "second hollow shaft" of an embodiment of the present invention) inserted into the outer periphery of the tip end portion 4 of the inner tube shaft 2.

The outer tube shaft 25 is an elongated hollow cylindrical member as in the inner tube shaft 2, and has an outer diameter identical to that of the base end portion 8 of the inner tube shaft 2. Further, the outer tube shaft 25 includes a plurality of slits 27 (each of which corresponds to the "second slit" of an embodiment of the present invention) formed on a surface thereof spirally in the counter-clockwise direction toward the tip end.

It is noted that the direction in which the slits 27 extend is a θ4 direction (which corresponds to the "second direction" according to an embodiment of the present invention. See Fig. 8.) relative to the longitudinal direction of the medical tube 23, and spirally opposite to the θ2 direction of the slits 6. Further, the outer tube shaft 25 has a lumen 25a into which the tip end portion 4 of the inner tube shaft 2 can be inserted.

Moreover, the outer tube shaft 25 may be made of a similar material as those of the outer tube shaft 5 and the outer tube shaft 15.

In the medical tube 23 according to the present embodiment, the spiral direction of the slits 27 formed on the outer tube shaft 25 is opposite to the spiral direction of the slits 6 formed on the inner tube shaft 2. Therefore, the outer tube shaft 25 or the inner tube shaft 2 can rotate in a tightening direction when a base end of the medical tube 23 is rotated in either the clockwise or counter-clockwise direction, leading to improved torquability of the medical tube 23.

Further, torquability can be secured by the integral formation of the base end portion 8 and the tip end portion 4 of the inner tube shaft 2. Moreover, flexibility can be improved by the presence of the slits 6 and the slits 27 formed on the tip end portion 4 of the inner tube shaft 2 and the outer tube shaft 25. Furthermore, the direction in which the slits 6 extend is configured to cross the direction in which the slits 17 extend. This can disperse stress over the inner tube shaft 2 and the outer tube shaft 25 without causing the medical tube 23 inoperable even when the medical tube 23 is curved, thereby preventing rapture of the medical tube 23.

It is noted that in the present embodiment, the slits 6 are configured so as to extend in the θ2 direction relative to the longitudinal direction of the medical tube 23, and the slits 27 are configured so as to extend in the θ4 direction perpendicular to the longitudinal direction of the medical tube 24, but the configuration shall not be limited to this embodiment. Similar effects can be obtained as long as the slits 6 and the slits 27 are formed in spirally opposite directions.

### Fourth embodiment

Next, the fourth embodiment of the present invention will be described. It is noted that the figures used for describing the present embodiment may also be drawn with exaggeration for clear understanding, and may not be scaled correctly.

Below, the fourth embodiment of the present invention will be described. The sensor guide wire has an overall structure similar to that shown in Fig. 1 and an inner tube shaft 2 similar to that shown in Fig. 4. Therefore, their descriptions will be omitted. The same symbols are assigned to portions common with the first embodiment, and their descriptions are omitted.

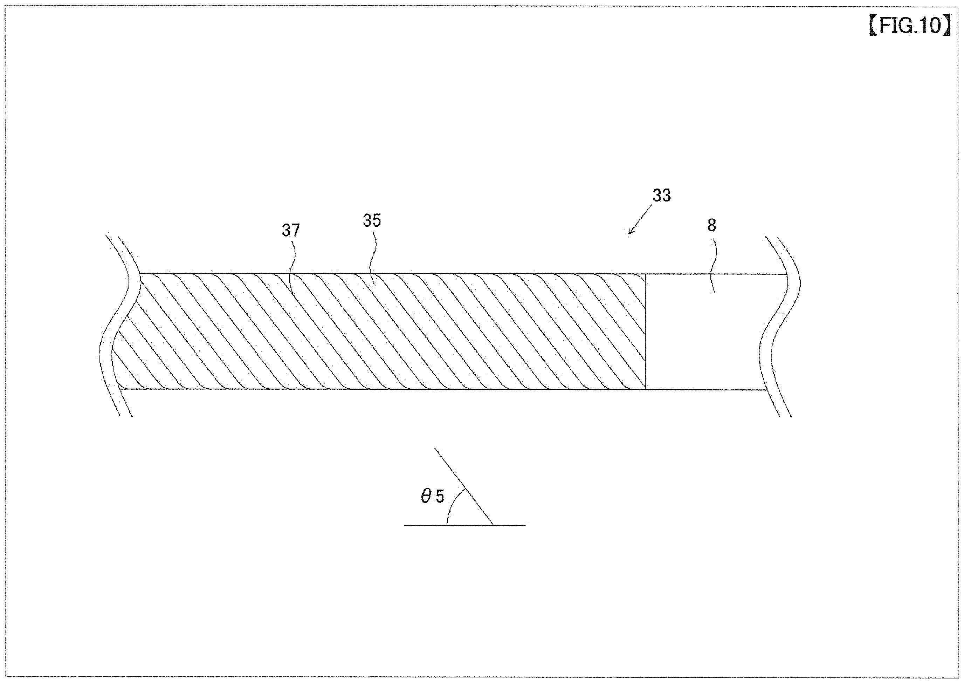

Fig. 10 shows an external view of a medical tube according to the fourth embodiment.

As shown in Fig. 10, a medical tube 33 includes the inner tube shaft 2 (which corresponds to the "first hollow shaft" according to an embodiment of the present invention) including the base end portion 8 and the tip end portion 4, the tip end portion 4 being formed in the tip end side of the base end portion 8; and a hollow twisted wire 35 (which corresponds to the "second hollow shaft" according to an embodiment of the present invention) including a single twisted element wire or multiple twisted element wires wound around the outer periphery of the tip end portion 4 of the inner tube shaft 2.

The outer diameter of the hollow twisted wire 35 is identical to that of the base end portion 8 of the inner tube shaft 2, and a plurality of depressed portions 37 (which correspond to the "second slit" according to an embodiment of the present invention) are formed spirally in the counter-clockwise direction toward the tip end on a surface of the hollow twisted wire 35.

It is noted that the direction in which the slits 37 extend is a θ5 direction (which corresponds to the "second direction" according to an embodiment of the present invention. See Fig. 10.) relative to the longitudinal direction of the medical tube 33, and different from the θ2 direction of the slits 6. Further, the hollow twisted wire 35 has a lumen (not shown) into which the tip end portion 4 of the inner tube shaft 2 can be inserted.

Moreover, the hollow twisted wire 35 may be made of a similar material as those of the inner tube shaft 2 and the outer tube shaft 5.

In the medical tube 33 according to the present embodiment, the hollow twisted wire 35 is formed with a single twisted element wire or multiple twisted element wires. This can further improve flexibility of the tip end portion of the medical tube 33.

Further, torquability can be secured by the integral formation of the base end portion 8 and the tip end portion 4 of the inner tube shaft 2. Moreover, flexibility can be improved by the presence of the slits 6 and the depressed portions 37 formed on the tip end portion 4 of the inner tube shaft 2 and the hollow twisted wire 35. Furthermore, the direction in which the slits 6 extend is configured to cross the direction in which the depressed portions 37 extend. This can disperse stress over the inner tube shaft 2 and the hollow twisted wire 35 without causing the medical tube 33 inoperable even when the medical tube 33 is curved, thereby preventing rapture of the medical tube 33.

It is noted that in the present embodiment, the slits 6 are configured so as to extend in the θ2 direction relative to the longitudinal direction of the medical tube 33, and the depressed portions 37 are configured so as to extend in the θ5 direction perpendicular to the longitudinal direction of the medical tube 33, but the configuration shall not be limited to this embodiment. Similar effects can be obtained as long as the slits 6 and the depressed portions 37 are formed spirally in directions where they cross each other.

### Fifth embodiment

Next, the fifth embodiment of the present invention will be described. It is noted that the figures used for describing the present embodiment may also be drawn with exaggeration for clear understanding, and may not be scaled correctly. In particular, the shapes of the slits described below are significantly enlarged for clear understanding.

Below, the fifth embodiment of the present invention will be described. The sensor guide wire has an overall structure similar to that shown in Fig. 1. Therefore, descriptions thereof will be omitted. The same symbols are assigned to portions common with the first embodiment, and their descriptions are omitted.

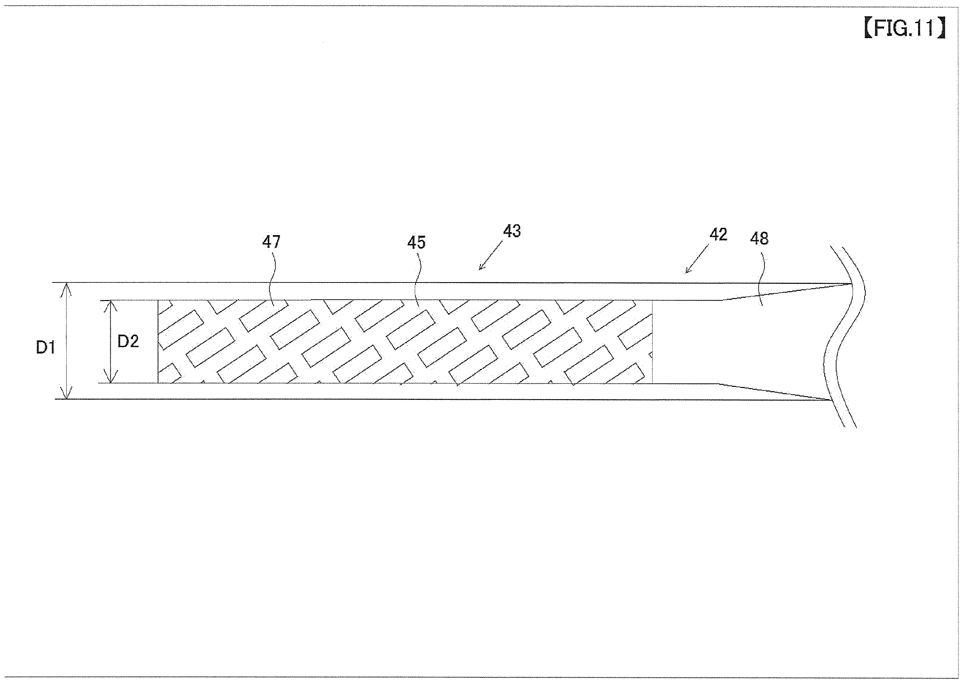

Fig. 11 shows an external view of a medical tube according to the fifth embodiment.

As shown in Fig. 11, a medical tube 43 includes an inner tube shaft 42 (which corresponds to the "first hollow shaft" according to an embodiment of the present invention) including a base end portion 48 and a tip end portion (not shown), the tip end portion being formed in the tip end side of the base end portion 48; and an outer tube shaft 45 (which corresponds to the "second hollow shaft" according to an embodiment of the present invention) inserted into the outer periphery of the tip end portion of the inner tube shaft 42.

The inner tube shaft 42 is an elongated hollow cylindrical member, and includes the base end portion 48 and the tip end portion (not shown), the tip end portion being formed in the tip end side of the base end portion 48. The tip end portion has an outer diameter smaller than that of the base end portion 48, and a plurality of slits 46 (not shown, each of which corresponds to the "first slit" according to an embodiment of the present invention, and is in a form similar to the slits 6) are formed spirally in the clockwise direction toward the tip end on a surface of the tip end portion. Further, the inner tube shaft 42 has a lumen in communication with the base end portion 48 and the tip end portion thereof.

It is noted that in the present embodiment, the direction in which the slits 46 extend is the θ2 direction (which corresponds to the "first direction" according to an embodiment of the present invention. See Fig. 4.).

The outer tube shaft 45 is an elongated hollow cylindrical member as in the inner tube shaft 42, and an outer diameter D2 thereof is smaller than an outer diameter D1 of the base end portion 48 of the inner tube shaft 42. Further, the outer tube shaft 45 includes a plurality of slits 47 (each of which corresponds to the "second slit" of an embodiment of the present invention) formed on a surface thereof spirally in the counter-clockwise direction toward the tip end.

It is noted that the direction in which the slits 47 extend is the θ4 direction (which corresponds to the "second direction" according to an embodiment of the present invention. See Fig. 8.) relative to the longitudinal direction of the medical tube 43, and spirally opposite to the θ2 direction of the slits 6. Moreover, the outer tube shaft 45 has a lumen (not shown) into which the tip end portion of the inner tube shaft 42 can be inserted.

Moreover, the inner tube shaft 42 and the outer tube shaft 45 may be made of similar materials as those of the inner tube shaft 2 and the outer tube shaft 5.

In the medical tube 43 according to the present embodiment, the outer diameter of the tip end of the outer tube shaft 45 in a state where the outer tube shaft 45 covers the outer periphery of the tip end portion of the inner tube shaft 42 is smaller than that of the base end portion of the inner tube shaft 42. This can further improve flexibility of the tip end portion of the medical tube 43.

Further, torquability can be secured by the integral formation of the base end portion 48 and the tip end portion of the inner tube shaft 42. Moreover, flexibility can be improved by the presence of the slits 46 and the slits 47 formed on the tip end portion of the inner tube shaft 42 and the outer tube shaft 45. Furthermore, the direction in which the slits 46 extend is configured to cross the direction in which the slits 47 extend. This can disperse stress over the inner tube shaft 42 and the outer tube shaft 45 without causing the medical tube 43 inoperable even when the medical tube 43 is curved, thereby preventing rapture of the medical tube 43.

It is noted that in the present embodiment, the slits 46 are configured so as to extend in the θ2 direction relative to the longitudinal direction of the medical tube 43, and the slits 47 are configured so as to extend in the θ4 direction relative to the longitudinal direction of the medical tube 43, but the configuration shall not be limited to this embodiment. Similar effects can be obtained as long as the slits 46 and the slits 47 are formed in directions where they cross each other.

### Sixth embodiment

Next, the sixth embodiment of the present invention will be described. It is noted that the figures used for describing the present embodiment may also be drawn with exaggeration for clear understanding, and may not be scaled correctly. In particular, the shapes of the slits described below are significantly enlarged for clear understanding.

Below, the sixth embodiment of the present invention will be described. The sensor guide wire has an overall structure similar to that shown in Fig. 1. Therefore, descriptions thereof will be omitted. The same symbols are assigned to portions common with the first embodiment, and their descriptions are omitted.

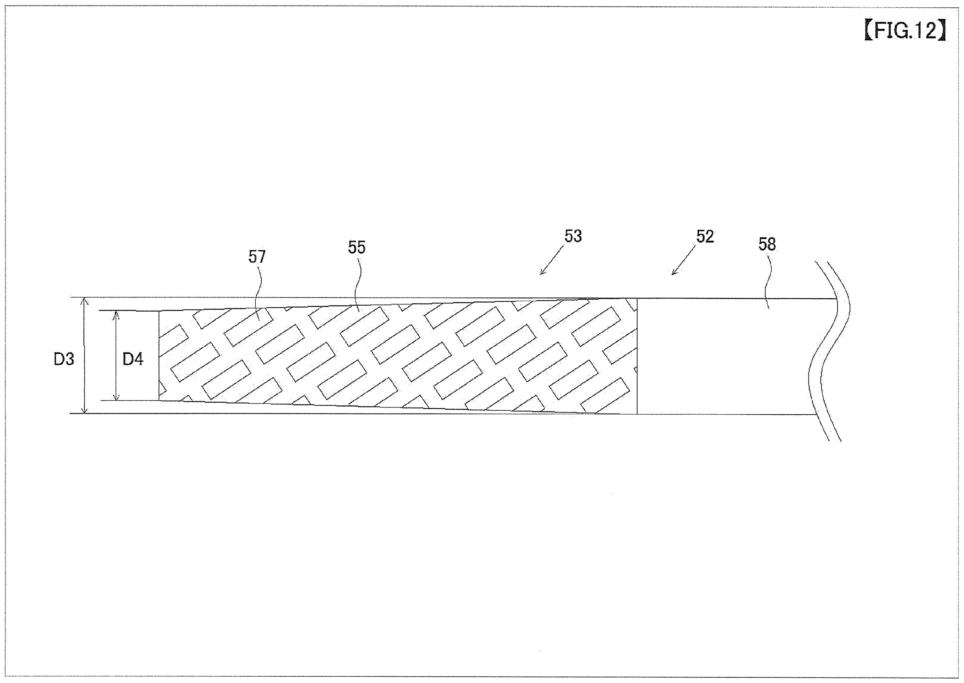

Fig. 12 shows an external view of a medical tube according to the sixth embodiment.

As shown in Fig. 12, a medical tube 53 includes an inner tube shaft 52 (which corresponds to the "first hollow shaft" according to an embodiment of the present invention) including a base end portion 58 and a tip end portion (not shown), the tip end portion being formed in the tip end side of the base end portion 58; and an outer tube shaft 55 (which corresponds to the "second hollow shaft" according to an embodiment of the present invention) inserted into the outer periphery of the tip end portion of the inner tube shaft 52.

The inner tube shaft 52 is an elongated hollow cylindrical member, and includes the base end portion 58 and the tip end portion (not shown), the tip end portion being formed in the tip end side of the base end portion 58. The tip end portion has an outer diameter smaller than that of the base end portion 58, and a plurality of slits 56 (not shown, each of which corresponds to the "first slit" according to an embodiment of the present invention, and are in a form similar to the slits 6) are formed spirally in the clockwise direction toward the tip end on a surface of the tip end portion. Further, the inner tube shaft 52 has a lumen in communication with the base end portion 58 and the tip end portion thereof.

It is noted that in the present embodiment, the direction in which the slits 56 extend is the θ2 direction (which corresponds to the "first direction" according to an embodiment of the present invention. See Fig. 4.).

The outer tube shaft 55, which is an elongated hollow member, has an external shape tapered toward the tip, and an outer diameter D4 of the tip end thereof is smaller than an outer diameter D3 of the base end portion 58 of the inner tube shaft 52. Further, the outer tube shaft 55 includes a plurality of slits 57 (each of which corresponds to the "second slit" of an embodiment of the present invention) formed on a surface thereof spirally in the counter-clockwise direction toward the tip end.

It is noted that the direction in which the slits 57 extend is the θ4 direction (which corresponds to the "second direction" according to an embodiment of the present invention. See Fig. 8.) relative to the longitudinal direction of the medical tube 53, and spirally opposite to the θ2 direction of the slits 6. Moreover, the outer tube shaft 55 has a lumen (not shown) into which the tip end portion of the inner tube shaft 52 can be inserted.

Moreover, the inner tube shaft 52 and the outer tube shaft 55 may be made of similar materials as those of the inner tube shaft 2 and the outer tube shaft 5.

In the medical tube 53 according to the present embodiment, the outer diameter of the tip end of the outer tube shaft 55 in a state where the outer tube shaft 55 covers the outer periphery of the tip end portion of the inner tube shaft 52 is smaller than that of the base end portion of the inner tube shaft 52. This can further improve flexibility of the tip end portion of the medical tube 53.

Further, torquability can be secured by the integral formation of the base end portion 58 and the tip end portion of the inner tube shaft 52. Moreover, flexibility can be improved by the presence of the slits 52 and the slits 57 formed on the tip end portion of the inner tube shaft 52 and the outer tube shaft 55. Furthermore, the direction in which the slits 56 extend is configured to cross the direction in which the slits 57 extend. This can disperse stress over the inner tube shaft 52 and the outer tube shaft 55 without causing the medical tube 53 inoperable even when the medical tube 53 is curved, thereby preventing rapture of the medical tube 53.

It is noted that in the present embodiment, the slits 56 are configured so as to extend in the θ2 direction relative to the longitudinal direction of the medical tube 43, and the slits 57 are configured so as to extend in the θ4 direction relative to the longitudinal direction of the medical tube 53, but the configuration shall not be limited to this embodiment. Similar effects can be obtained as long as the slits 56 and the slits 57 are formed in directions where they cross each other.

The medical tubes according to the various embodiments of the present invention are described above, but the present invention shall not be limited to these. Various alterations may be made without departing from the spirit of the present invention.

For example, the shapes of the slits in the various embodiments are drawn with exaggeration for illustrative purposes, but the shapes shall not be limited to these. The width and length of a slit may be appropriately selected.

Further, the wire diameter of an elemental wire for the hollow twisted wire 35 according to the fourth embodiment may also be selected appropriately.

Moreover, a plurality of slits is configured so as to extend spirally on a surface of the outer peripheral of an inner tube shaft and/or an outer tube shaft, but the configuration shall not be limited to these. For example, a configuration (spiral cut) may be used in which a single slit is formed so as to extend spirally.

### DESCRIPTION OF SYMBOLS

- 1: Sensor guide wire
- 2, 42, 52: Inner tube shaft
- 3, 13, 23, 33, 43, 53: Medical tube
- 4: Tip end portion
- 5, 15, 25, 45, 55: Outer tube shaft
- 4a, 5a, 15a, 25a: Lumen
- 6, 7, 17, 27, 37, 47, 57: Slit
- 8, 48, 58: Base end portion
- 9: Opening
- 35: Hollow twisted wire
- 37: Depressed portion

## Claims

1. A medical tube, comprising:
a first follow shaft including a base end portion and a tip end portion, the tip end portion being formed in a tip end side of the base end portion and having an outer diameter smaller than that of the base end portion and including a first slit formed on a surface thereof, the first slit extending in a first direction; and
a second hollow shaft for covering an outer periphery of the tip end portion of the first hollow shaft and including a second slit formed on a surface thereof, the second slit extending in a second direction crossing the first direction.

2. The medical tube according to claim 1, wherein the first slit formed on the first hollow shaft and the second slit formed on the second hollow shaft extend spirally.

3. The medical tube according to claim 2, wherein a spiral direction of the second slit formed on the second hollow shaft is opposite to a spiral direction of the first slit formed on the first hollow shaft.

4. The medical tube according to claim 2 or 3, wherein the second hollow shaft comprises a hollow twisted wire, the follow twisted wire comprising a single twisted elemental wire or multiple twisted elemental wires.

5. The medical tube according to any one of claims 1 to 4, wherein an outer diameter of a tip end of the second hollow shaft in a state where the second hollow shaft covers the outer periphery of the tip end portion of the first hollow shaft is smaller than that of the base end portion of the first hollow shaft.
